# EUROPÄISCHE PATENTSCHRIFT

(11) **EP 0 385 423 B1**
(45) Veröffentlichungstag und Bekanntmachung des Hinweises auf die Patenterteilung: **08.02.1995**
(21) Anmeldenummer: 90103870.3
(22) Anmeldetag: 28.02.1990
(51) Int. Cl.: C07B 57/00, C07D 471/04, A61K 31/44

(54) **Verfahren zur Herstellung der optischen Isomeren von 1,4-Dihydro-5-isopropoxy-2-methyl-4-(2-trifluormethylphenyl)-1,6-naphthyridin-3-carbonsäure-ethyl-ester und 1,4-Dihydro-5-isopropoxy-2-methyl-4-(2-trifluormethylphenyl)-1,6-naphthyridin-3-carbonsäure- 2-(N-methyl-N-phenylmethylamino)ethyl ester**
Process for the preparation of the optical isomers of 1,4-dihydro-5-isopropoxy-2-methyl-4-(2-trifluoromethyl phenyl)-1,6-naphthyridine-3-carboxylic-acid ethyl ester and 1,4-dihydro-5-isopropoxy-2-methyl-4-(2-trifluormethyl phenyl)-1,6-naphthyridine-3-carboxylic-acid-2-(N-methyl-N-phenyl methyl amino) ethyl ester
Procédé de préparation des isomères optiques du 1,4-dihydro-5-isopropoxy-2-méthyl-4-(2-trifluorométhyl phényl)-1,6-naphtyridine-3-carboxylate d'éthyle et du 1,4-dihydro-5-isopropoxy-2-méthyl-4-(2-trifluorométhyl phényl)-1,6-naphtyridine-2-(N-méthyl-N-phénylméthylamino)3-carboxylate d'éthyle

(30) Priorität: 01.03.1989 DE 3906460
(43) Veröffentlichungstag der Anmeldung: 05.09.1990
(73) Patentinhaber: GÖDECKE AKTIENGESELLSCHAFT, D-10587 Berlin (DE)
(72) Erfinder: Herrmann, Wolfgang, Dr., D-7802 Merzhausen (DE); Kleinschroth, Jürgen, Dr., D-7819 Denzlingen (DE); Steiner, Klaus, Dr., D-7808 Waldkirch (DE)

(56) Entgegenhaltungen:
- EP-A- 0 013 561
- DE-A- 3 431 303

## Beschreibung

Die Naphthyridin-Derivate (±)-1,4-Dihydro-5-isopropoxy-2-methyl-4-(2-trifluormethylphenyl)-1,6-naphthyridin-3-carbonsäure-ethylester (Formel I) und (±)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester (Formel II) 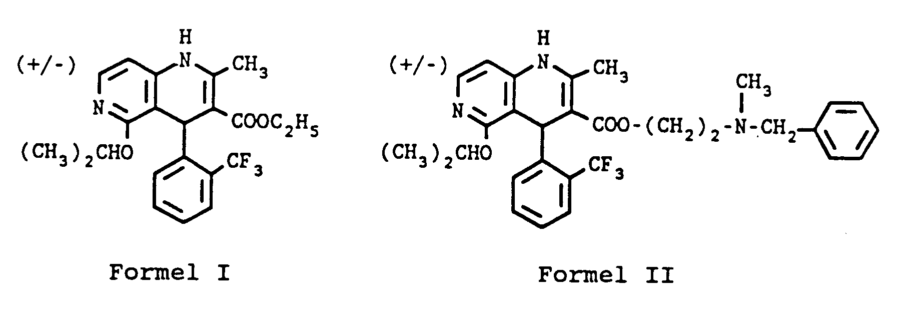
sind aus der DE-A-34 31 303 bekannt und pharmakologisch als hochwirksame Calcium-Antagonisten identifiziert. Beide Wirkstoffe sind chirale Verbindungen und die bei der Synthese entstehenden schwach basischen racemischen Verbindungen konnten bisher nicht direkt über diastereomere Salze mit optisch aktiven Hilfsstoffen in die enantiomeren Formen getrennt werden. Auch eine Trennung der entsprechenden Naphthyridin-3-carbonsäure in die optischen Antipoden durch fraktionierte Kristallisation mit optisch aktiven Basen oder Säuren gelang bisher nicht. Die Trennung der Verbindungen der Formeln I und II in die optischen Antipoden ist jedoch erwünscht, denn in pharmakologischen Vergleichsversuchen stellte sich heraus, daß die rechtsdrehenden Enantiomeren wesentlich stärker wirksam sind als die Racemate.

Aufgabe der Erfindung ist es daher, ein ökonomisches und technisch durchführbares Verfahren zu entwickeln, um eine Synthesezwischenstufe oder ein Derivat dieser Verbindungen in die Enantiomeren aufzutrennen, und diese dann in die optisch aktiven Endverbindungen zu überführen.

Eine Möglichkeit der Synthese der enantiomeren Formen besteht im Gramm-Maßstab über die diastereomeren 1-Phenylethylester. Das hierfür benötigte S-(-)-1-Phenylethanol ist allerdings sehr teuer. Nach Abtrennung des rechtsdrehenden Diastereomeren wird der 1-Phenylethylester hydrogenolytisch gespalten und das eingesetzte S-(-)-1-Phenylethanol kann somit nicht mehr für weitere Trennungen verwendet werden.

Überraschenderweise wurde nun gefunden, daß der Benzylester dieser Naphthyridin-3-carbonsäuren mit optisch aktiver O,O′-Dibenzoyl-weinsäure in verschiedenen Lösungsmitteln, vorzugsweise Alkoholen, gut kristallisierende diastereomere Salze bildet, welche sich durch fraktionierte Kristallisation trennen lassen. Die chiralen Benzylester können dann leicht und mit guter Ausbeute entweder durch Umesterung direkt in die gewünschten Ester umgewandelt werden, oder es kann zuerst der Benzylester hydrogenolytisch zur Carbonsäure gespalten und diese über das Säurechlorid und dessen Umsetzung mit dem entsprechenden Alkohol in die gewünschten chiralen Endprodukte überführt werden. In beiden Fällen findet keine Racemisierung statt.

Die Lösung der Aufgabe besteht daher darin, daß der als Zwischenprodukt leicht herstellbare racemische Benzylester (±)-1,4-Dihydro-5-isopropoxy-2-methyl-4-(2-trifluormethylphenyl)-1,6-naphthyridin-3-carbonsäure-benzylester mit Hilfe von optisch aktiver (+)- oder (-)-O,O′-Dibenzoyl-weinsäure (DBTA) über die fraktionierte Kristallisation der diastereomeren Salze in das rechts drehende und das linksdrehende Enantiomere aufgetrennt wird. Die hierfür benötigte (+)-O,O′-Dibenzoyl-D-weinsäure bzw. (-)-O,O′-Dibenzoyl-L-weinsäure sind wesentlich preisgünstiger. Außerdem können die beiden Hilfssäuren nach erfolgter Trennung zurückgewonnen und erneut eingesetzt werden.

Auf entsprechende Weise können auch die linksdrehenden Enantiomeren hergestellt werden.

Mit dem erfindungsgemäßen Verfahren ist es möglich, die gewünschten enantiomerenreinen Calcium-Antagonisten in größerer Menge und in wirtschaftlicher Form herzustellen. Das Verfahren führt zu einer deutlichen Kostenersparnis durch geringere Kosten für die Hilfreagenzien zur Racemattrennung, wobei das Hilfreagenz O,O′-Dibenzoyl-weinsäure im Gegensatz zu 1-Phenylethanol sogar noch wiederholt eingesetzt werden kann. Außerdem werden durch das erfindungsgemäße Verfahren mindestens 3, gegebenenfalls sogar 5 Synthesestufen eingespart, wie an dem Syntheseschema I aufgezeigt wird. Eine wirtschaftliche Nutzung der enantiomeren Verbindungen wird daher durch die vorliegende Erfindung überhaupt erst ermöglicht.

(+)-1,4-Dihydro-5-isopropoxy-2-methyl-4-(2-trifluormethylphenyl)-1,6-naphthyridin-3-carbonsäure-ethylester und (-)-1,4-Dihydro-5-isopropoxy-2-methyl-4-(2-trifluormethylphenyl)-1,6-naphthyridin-3-carbonsäure-ethylester werden gemäß Reaktionsschema I hergestellt, indem
A) aus (±)-1,4-Dihydro-5-isopropoxy-2-methyl-4-(2-trifluormethylphenyl)-1,6-naphthyridin-3-carbonsäure-ethylester durch Umesterung (±)-1,4-Dihydro-5-isopropoxy-2-methyl-4-(2-trifluormethylphenyl)-1,6-naphthyridin-3-carbonsäure-benzylester hergestellt wird,
B) aus diesem durch Salzbildung mit optisch aktiver (+)- oder (-)-Dibenzoyl-weinsäure und fraktionierter Kristallisation der diastereomeren Salze aus geeigneten Lösungsmitteln und anschließender Freisetzung der Basen die enantiomeren Benzylester (+)-1,4-Dihydro-5-isopropoxy-2-methyl-4-(2-trifluormethylphenyl)-1,6-naphthyridin-3-carbonsäure-benzylester und (-)-1,4-Dihydro-5-isopropoxy-2-methyl-4-(2-trifluormethylphenyl)-1,6-naphthyridin-3-carbonsäure-benzylester hergestellt werden und aus diesen entweder
   a) durch katalytische Hydrierung die entsprechenden enantiomeren Carbonsäuren hergestellt und diese nach Überführung in das Säurechlorid anschließend mit Ethanol verestert werden oder
   b) die gewünschten optisch aktiven Verbindungen direkt durch Umesterung mit Ethanol hergestellt werden.

In analoger Weise werden (+)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester und (-)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester hergestellt, indem für die Veresterung in Stufe B), a) oder die Umesterung in Stufe B) b) 2-(N-Benzyl-N-methylamino)ethanol eingesetzt wird.

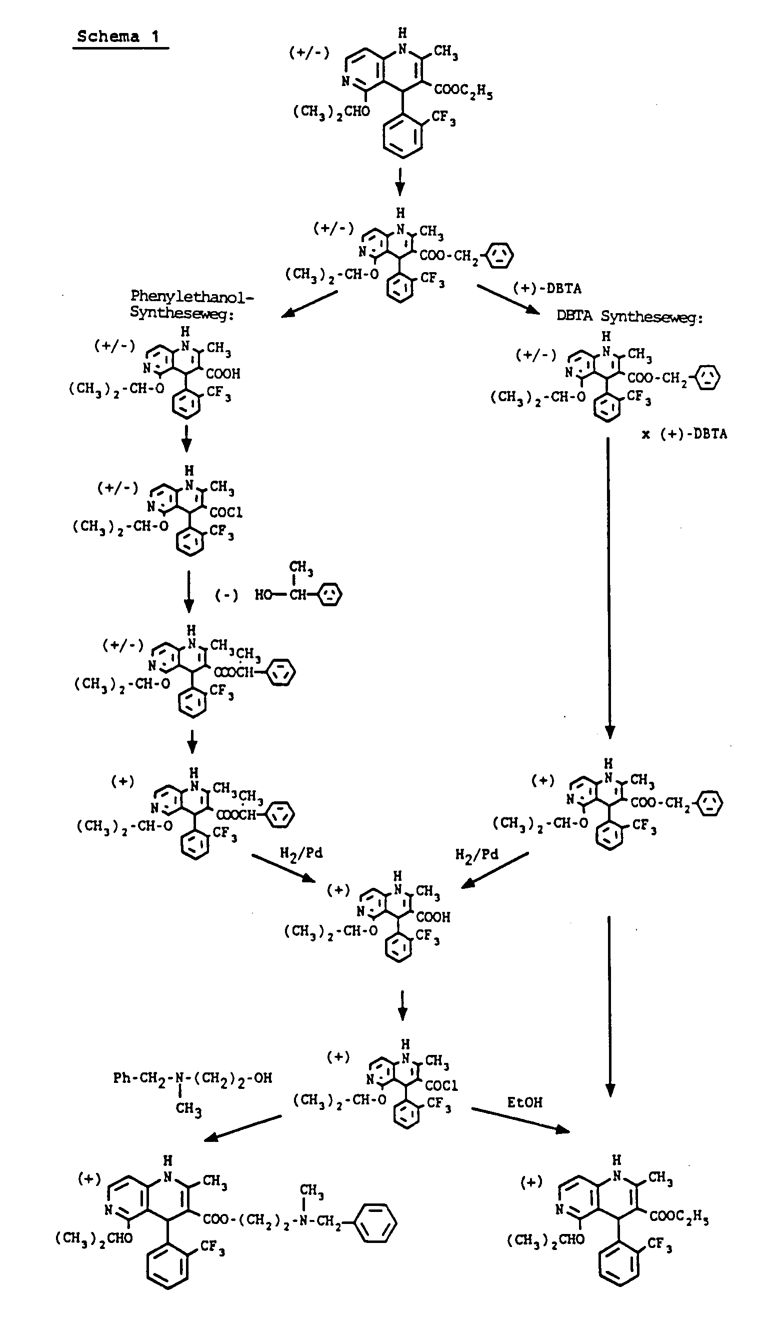

Die Racemattrennung wird durch die folgenden Ausführungsbeispiele belegt:

### Beispiele

### Beispiel 1

### (±)-1,4-Dihydro-5-isopropoxy-2-methyl-4-(2-trifluormethylphenyl)-1,6-naphthyridin-3-carbonsäure-benzylester

In einem 500 ml Vierhalsrundkolben, ausgestattet mit einem Rührer, Tropftrichter, Rückflußkühler mit Gasableitung, einem Thermometer und einer Gaseinleitung werden 100 ml Benzylalkohol vorgelegt und unter Schutzgasspülung 0,937g (0,0312 mol) Natriumhydrid 80 %ig in Öl eingetragen. Die entstehende Suspension läßt man 30 min nachrühren. Anschließend läßt man eine Lösung aus 52,55 g (0,124 mol) (±)-1,4-Dihydro-5-isopropoxy-2-methyl-4-(2-trifluormethylphenyl)-1,6-naphthyridin-3-carbonsäure-ethylester in 200 ml Benzylalkohol zulaufen. Danach wird 15 h bei 120°C gerührt. Das Reaktionsgemisch wird nach Abkühlen auf Raumtemperatur mit 1 l Wasser und 400 ml Dichlormethan verrührt. Nach dem Trennen der Phasen wird die wässrige Phase nochmals mit 300 ml Dichlormethan ausgerührt. Die Dichlormethanphasen werden vereinigt und mit 2 x 300 ml Wasser gewaschen. Die Dichlormethanphase wird über Natriumsulfat getrocknet. Nach dem Filtrieren wird das Dichlormethan am Rotationsverdampfer abdestilliert, anschließend wird der Benzylalkohol im Ölpumpenvakuum abdestilliert (54-65°C/3-5 mm). Der Destillationsrückstand wird in 600 ml heißem Cyclohexan gelöst. Die Lösung wird unter Rühren auf 0°C gekühlt. Der ausgefallene Niederschlag wird abgesaugt und mit 100 ml n-Hexan nachgewaschen. Der Nutschkuchen wird bei 80°C getrocknet. Ausbeute: 43,9 g = 72,8 %. vom Schmp. 131°C bei einer Reinheit von 99,4 rel%).

### Beispiel 2

### (+)-1,4-Dihydro-5-isopropoxy-2-methyl-4-(2-trifluormethylphenyl)-1,6-naphthyridin-3-carbonsäure-benzylester

1,93 g (0,004 mol) (±)-1,4-Dihydro-5-isopropoxy-2-methyl-4-(2-trifluormethylphenyl)-1,6-naphthyridin-3-carbonsäure-benzylester (Beispiel 1) und 1,50 g (0,004 mol) (+)-O,O′-Dibenzoyl-D-weinsäure Monohydrat werden in 20,6 ml heißem Isopropylalkohol gelöst. Die Lösung läßt man mehrere Tage bei 8°C stehen. Das ausgefallene Produkt wird abgesaugt und im Vakuumtrockenschrank bei 40°C getrocknet. (588 mg; Fp: 159-162°C; [α]_{D} = + 82,6° (c = 1/MeOH).

Zur Freisetzung der Base wird das Salz in 19 ml 1N Natronlauge suspendiert und mit 5 x 7 ml Toluol extrahiert. Die vereinigten organischen Phasen werden mit 1 x 7 ml 1N Natronlauge und 3 x 7 ml Wasser gewaschen. Die organische Phase wird über Natriumsulfat getrocknet und anschließend am Rotationsverdampfer bis zur Trockne eingeengt. Der feste Rückstand wird aus 20 ml n-Hexan umkristallisiert. Das Kristallisat wird im Vakuumtrockenschrank bei 40°C getrocknet. (Ausbeute: 300 mg = 31,08 %; Fp: 110-111°C; [α]_{D} = + 39,3° (c = 1/CH₂Cl₂).

### Beispiel 3

### (-)-1,4-Dihydro-5-isopropoxy-2-methyl-4-(2-trifluormethylphenyl)-1,6-naphthyridin-3-carbonsäure-benzylester

9,65 g (0,02 mol) (±)-1,4-Dihydro-5-isopropoxy-2-methyl-4-(2-trifluormethylphenyl)-1,6-naphthyridin-3-carbonsäure-benzylester (Beispiel 1) und 15,04 g (0,04 mol) (-)-O,O′-Dibenzoyl-L-weinsäure Monohydrat werden in einer heißen Mischung von 100 ml Ethanol und 70 ml Wasser gelöst. Die Lösung läßt man langsam auf 20°C abkühlen. Das ausgefallene Produkt wird abgesaugt und im Vakuumtrockenschrank bei 40°C getrocknet.

Zur Freisetzung der Base wird das Salz in 19 ml 1N Natronlauge suspendiert und mit 5 x 7 ml Toluol extrahiert. Die vereinigten organischen Phasen werden mit 1 x 7 ml 1N Natronlauge und 3 x 7 ml Wasser gewaschen. Die organische Phase wird über Natriumsulfat getrocknet und anschließend am Rotationsverdampfer bis zur Trockne eingeengt. Der feste Rückstand wird aus 20 ml n-Hexan umkristallisiert. Das Kristallisat wird im Vakuumtrockenschrank bei 40°C getrocknet. (Fp: 110°C; [α]D = + 39,3° (c = 1/CH₂Cl₂); Ausbeute 1,3 g = 27,7%).

### Beispiel 4

### (+)-1,4-Dihydro-5-isopropoxy-2-methyl-4-(2-trifluormethylphenyl)-1,6-naphthyridin-3-carbonsäure

1,6 g (0,00331 mol) (+)-1,4-Dihydro-5-isopropoxy-2-methyl-4-(2-trifluormethylphenyl)-1,6-naphthyridin-3-carbonsäure-benzylester werden in 32 ml Ethanol aufgenommen und drucklos bei 20°C in Gegenwart von 0,64 g Pd/C 10%ig (50% feucht) hydriert. Der Katalysator wird abfiltriert und das Filtrat am Rotationsverdamfer bei 35°C bis zur Trockne eingeengt. Der feste Rückstand wird aus 10 ml Diisopropylether umkristallisiert. Das Produkt wird im Vakuumtrockenschrank bei 40°C getrocknet. (Ausbeute: 1,04 g = 80,3%).

### Beispiel 5

### (+)-1,4-Dihydro-5-isopropoxy-2-methyl-4-(2-trifluormethylphenyl)-1,6-naphthyridin-3-carbonsäure-ethylester

Zu einer auf 3°C gekühlten Lösung von 0,5 ml abs. Dimethylformamid in 3 ml abs. Dioxan werden 31,4 mg Oxalylchlorid zugegeben und 40 min bei 0-5°C gerührt. Nach Zugabe von 85 mg (+)-1,4-Dihydro-5-isopropoxy-2-methyl-4-(2-trifluormethylphenyl)-1,6-naphthyridin-3-carbonsäure, gelöst in 1 ml abs. Dioxan wird die Kühlung entfernt und 2,5 h gerührt. Nach der Zugabe von 2 ml Ethanol wird das Ganze zunächst 2 h bei Raumtemperatur, danach 30 min bei 40-50°C gerührt. Danach wird das Reaktionsgemisch mit 30 ml 2N Natriumcarbonatlösung und 10 ml Dichloromethan verrührt. Die organische Phase wird abgetrennt und die wässrige Phase mit 3 x 10 ml Dichlormethan nachextrahiert. Die vereinigten organischen Phasen werden über Natriumsulfat getrocknet und bis zur Trockne eingeengt. Der Rückstand wird über Kieselgel mit n-Hexan/Essigester (1:1) als Elutionsmittel gereinigt und anschließend aus n-Hexan umkristallisiert. Ausbeute: 32 mg (38,5 %); FP: 130°C; [α]_{D} = + 105° (c = 0,5/CH₂Cl₂).

### Beispiel 6

### (+)-1,4-Dihydro-5-isopropoxy-2-methyl-4-(2-trifluormethylphenyl)-1,6-naphthyridin-3-carbonsäure-ethylester

1,44 g (+)-1,4-Dihydro-5-isopropoxy-2-methyl-4-(2-trifluormethylphenyl)-1,6-naphthyridin-3-carbonsäure-benzylester werden in eine Lösung von 100 mg Natrium in 90 ml abs. Ethanol eingetragen und 20 h unter Rückfluß gekocht. Das Reaktionsgemisch wird eingeengt, der Rückstand in 50 ml Dichlormethan aufgenommen und mit 3 x 20 ml Wasser extrahiert. Die organische Phase wird über Natriumsulfat getrocknet und danach bis zur Trockne eingeengt. Der Rückstand wird aus 20 ml n-Hexan umkristallisiert. Der Nutschkuchen wird im Vakuumtrockenschrank bei 30°C bis zur Gewichtskonstanz getrocknet. Ausbeute: 850 mg (67,38%); FP: 130°C; [α]_{D} = + 110°C (c = 0,5/CH₂Cl₂).

### Beispiel 7

### (-)-1,4-Dihydro-5-isopropoxy-2-methyl-4-(2-trifluormethylphenyl)-1,6-naphthyridin-3-carbonsäure

17 g (0,0352 mol) (-)-1,4-Dihydro-5-isopropoxy-2-methyl-4-(2-trifluormethylphenyl)-1,6-naphthyridin-3-carbonsäure-benzylester werden in 340 ml Ethanol aufgenommen und drucklos bei 20°C in Gegenwart von 6,8 g Pd/C 10%ig (50% feucht) hydriert. Der Katalysator wird abfiltriert und das Filtrat am Rotationsverdampfer bei 35°C bis zur Trockne eingeengt. (Ausbeute: 15,1 g = 89,2%).

### Beispiel 8

### (-)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester

Zu einer auf 0°C gekühlten Lösung von 11,3 ml abs. Dimethylformamid in 220 ml abs. Essigester wird eine Lösung aus 6,29 g Oxalylchlorid in 73 ml Essigester zugegeben und 15 min bei 0-5°C gerührt. Nach Zugabe von 12,8 g (-)-1,4-Dihydro-5-isopropoxy-2-methyl-4-(2-trifluormethylphenyl)-1,6-naphthyridin-3-carbonsäure werden 3,9 g Pyridin, gelöst in 73 ml Essigester zugetropft und 60 min bei 0°C gerührt. Nach der Zugabe von 10,26 g 2-(N-Benzyl-N-methylamino)-ethanol, gelöst in 3,03 g Pyridin und 146 ml Methylenchlorid wird das Ganze 1 h bei 5-10°C gerührt. Danach wird das Reaktionsgemisch mit 3 x 140 ml Wasser und mit 2 x 140 ml 1 N Natronlauge verrührt. Die organische Phase wird über Natriumsulfat getrocknet und bis zur Trockne eingeengt. Der ölige Rückstand wird über Kieselgel mit Toluol/Essigester (7:3) als Elutionsmittel gereinigt. Nach dem Abdestillieren des Elutionsmittels bleibt eine ölige Substanz zurück. Das Öl wird zusammen mit 2,52 g Fumarsäure in 350 ml Essigester unter Rückflußkochen gelöst. Nach Filtration wird auf ca. 50 ml eingeengt. Das hierbei ausfallende Produkt wird abgesaugt, in 10 ml Toluol aufgeschlämmt und mit 5 ml 1m Ammoniaklösung verrührt. Die Toluolphase wird mit 2 x 5 ml Wasser gewaschen. Die organische Phase wird nach dem Trocknen über Natriumsulfat bis zur Trockne eingeengt. Es bleibt eine ölige Substanz zurück. Ausbeute: 5,3 g = 32 %; [α]D = - 40,8° (c = 1/CH₂Cl₂).

### Beispiel 9

### (-)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester

21,05 g 2-(N-Benzyl-N-methylamino)ethanol werden in 42 ml Toluol gelöst und unter Stickstoff mit 0,74 g Natriumhydrid (80%ig) versetzt. Nach 30 minütigem Rühren wird eine Lösung von 8,42 g (0,0174 mol) (-)-1,4-Dihydro-5-isopropoxy-2-methyl-4-(2-trifluormethylphenyl)-1,6-naphthyridin-3-carbonsäure-benzylester zugetropft. Das Reaktionsgemisch wird unter Stickstoff und Feuchtigkeitsausschluß 4 h unter Rückfluß gekocht. Danach wird das Reaktiongemisch mit 2 x 50 ml 2 N Essigsäure extrahiert. Die organische Phase wird über Natriumsulfat getrocknet und bis zur Trockne eingeengt. Der Rückstand wird über Kieselgel mit Toluol/Essigester (7:3) als Elutionsmittel gereinigt. Nach dem Abdestillieren des Elutionsmittels bleibt eine ölige Substanz zurück. Das Öl wird zusammen mit 1,28 g Fumarsäure in 180 ml Essigester unter Rückflußkochen gelöst. Nach Filtration wird auf ca. 20 ml eingeengt. Das hierbei ausfallende Produkt wird abgesaugt, in 14 ml Toluol aufgeschlämmt und mit 3 ml 1m Ammoniaklösung verrührt. Die Toluolphase wird mit 2 x 5 ml Wasser gewaschen. Die organische Phase wird nach dem Trocknen über Natriumsulfat bis zur Trockne eingeengt. Es bleibt eine ölige Substanz zurück. Ausbeute: 0,8 g = 8,5 %, [α]D = - 40,8° (c = 1/CH₂Cl₂).

### Beispiel 10

### (+)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester

Zu einer auf 0°C gekühlten Lösung von 248 mg abs. Dimethylformamid in 4,1 ml abs. Essigester wird eine Lösung aus 138 mg Oxalylchlorid in 2 ml Essigester zugegeben und 15 min bei 0-5°C gerührt. Nach Zugabe von 270 mg (+)-1,4-Dihydro-5-isopropoxy-2-methyl-4-(2-trifluormethylphenyl)-1,6-naphthyridin-3-carbonsäure werden 0,09 ml Pyridin, gelöst in 2 ml Essigester zugetropft und 60 min bei 0°C gerührt. Nach der Zugabe von 224 mg 2-(N-Benzyl-N-methylamino)-ethanol, gelöst in 4 ml Methylenchlorid wird das Ganze 1 h bei 5-10°C gerührt. Danach wird das Reaktionsgemisch mit 3 x 10 ml Wasser und mit 2 x 10 ml 1 N Natronlauge verrührt. Die organische Phase wird über Natriumsulfat getrocknet und bis zur Trockne eingeengt. Das Öl wird zusammen mit 78 mg Fumarsäure in 5 ml Essigester unter Rückflußkochen gelöst. Nach Filtration wird auf ca. 2 ml eingeengt. Das hierbei ausfallende Produkt wird abgesaugt, in 5 ml Methylenchlorid aufgeschlämmt und mit 5 ml 1m Ammoniaklösung verrührt. Die organische Phase wird mit 2 x 5 ml Wasser gewaschen und nach dem Trocknen über Natriumsulfat bis zur Trockne eingeengt. Es bleibt eine ölige Substanz zurück. Ausbeute: 140 mg = 38 %, [α]D = + 43,8° (c = 1/CH₂Cl₂).

## Patentansprüche

1. Verfahren zur Herstellung von (+)-1,4-Dihydro-5-isopropoxy-2-methyl-4-(2-trifluormethylphenyl)-1,6-naphthyridin-3-carbonsäure-ethylester und (-)-1,4-Dihydro-5-isopropoxy-2-methyl-4-(2-trifluormethylphenyl)-1,6-naphthyridin-3-carbonsäure-ethylester, dadurch gekennzeichnet, daß
A) aus (±)-1,4-Dihydro-5-isopropoxy-2-methyl-4-(2-trifluormethylphenyl)-1,6-naphthyridin-3-carbonsäureethylester durch Umesterung (±)-1,4-Dihydro-5-isopropoxy-2-methyl-4-(2-trifluormethylphenyl)-1,6-naphthyridin-3-carbonsäure-benzylester hergestellt wird,
B) aus diesem durch Salzbildung mit optisch aktiver (+)- oder (-)-Dibenzoyl-weinsäure und fraktionierter Kristallisation der diastereomeren Salze aus geeigneten Lösungsmitteln und anschließender Freisetzung der Basen die enantiomeren Benzylester (+)-1,4-Dihydro-5-isopropoxy-2-methyl-4-(2-trifluormethylphenyl)-1,6-naphthyridin-3-carbonsäurebenzylester und (-)-1,4-Dihydro-5-isopropoxy-2-methyl-4-(2-trifluormethylphenyl)-1,6-naphthyridin-3-carbonsäurebenzylester hergestellt werden und aus diesen entweder
a) durch katalytische Hydrierung die entsprechenden enantiomeren Carbonsäuren hergestellt und diese nach Überführung in das Säurechlorid anschließend mit Ethanol verestert werden oder
b) die gewünschten optisch aktiven Verbindungen direkt durch Umesterung mit Ethanol hergestellt werden.

2. Verfahren gemäß Anspruch 1 zur Herstellung von (+)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester und (-)-1,4-Dihydro-5-isopropoxy-2-methyl-4-(2-trifluormethylphenyl)-1,6-naphthyridin-3-carbonsäure-[2-(N-methyl-N-phenylmethylamino)ethyl]ester dadurch gekennzeichnet, daß für die Veresterung in Stufe B), a) oder die Umesterung in Stufe B) b) 2-(N-Benzyl-N-methylamino)ethanol eingesetzt wird.

## Claims

1. A process for the preparation of ethyl (+)-1,4-dihydro-5-isopropoxy-2-methyl-4-(2-trifluoromethylphenyl)-1,6-naphthyridine-3-carboxylate and ethyl (-)-1,4-dihydro-5- isopropoxy-2-methyl-4-(2-trifluoromethylphenyl)-1,6-naphthyridine-3-carboxylate, characterized in that
A) benzyl (±)-1,4-dihydro-5-isopropoxy-2-methyl-4-(2-trifluoromethylphenyl)-1,6-naphthyridine-3-carboxylate is prepared from ethyl (±)-1,4-dihydro-5-isopropoxy-2-methyl-4-(2- trifluoromethylphenyl)-1,6-naphthyridine-3-carboxylate by transesterification,
B) the enantiomeric benzyl esters, benzyl (+)-1,4-dihydro-5-isopropoxy-2-methyl-4-(2-trifluoromethylphenyl)-1,6-naphthyridine-3-carboxylate and benzyl (-)-1,4-dihydro-5-isopropoxy-2-methyl-4-(2-trifluoromethylphenyl)-1,6-naphthyridine-3-carboxylate, are prepared from said racemic benzyl ester by salification with optically active (+)- or (-)- dibenzoyl-tartaric acid and fractional crystallization of the diastereoisomeric salts from suitable solvents, followed by liberation of the bases, and either
a) the corresponding enantiomeric carboxylic acids are prepared from said enantiomeric benzyl esters by catalytic hydrogenation and said acids are converted to the acid chloride and then esterified with ethanol, or
b) the desired optically active compounds are prepared directly from said enantiomeric benzyl esters by transesterification with ethanol.

2. A process according to Claim 1 for the preparation of 2-(N-methyl-N-phenylmethylamino)ethyl (+)-1,4-dihydro-5- isopropoxy-2-methyl-4-(2-trifluoromethylphenyl)-1,6-naphthyridine-3-carboxylate and 2-(N-methyl-N-phenylmethylamino)ethyl (-)-1,4-dihydro-5-isopropoxy-2-methyl-4-(2-trifluoromethylphenyl)-1,6-naphthyridine-3-carboxylate, characterized in that 2-(N-benzyl-N-methylamino)ethanol is used for the esterification in step B) a) or the transesterification in step B) b).

## Revendications

1. Procédé d'obtention de (+)-1,4-dihydro-5-isopropoxy-2-méthyl-4-(2-trifluorométhyl-phényl)-1,6-naphtyridine-3-carboxylate d'éthyle et (-)-1,4-dihydro-5-isopropoxy-2-méthyl-4-(2-trifluorométhyl-phényl)-1,6-naphtyridine-3-carboxylate d'éthyle caractérisé en ce que:
A) à partir de (±)-1,4-dihydro-5-isopropoxy-2-méthyl-4-(2-trifluorométhyl-phényl)-1,6-naphtyridine-3-carbo xylate d'éthyle par transestérification, on prépare du (±)-1,4-dihydro-5-isopropoxy-2-méthyl-4-(2-tri fluorométhyl-phényl)-1,6-naphtyridine-3-carboxylate de benzyle;
B) à partir de ce produit, par formation de sels par réaction avec un acide (±)-dibenzoyltartrique opti quement actif et cristallisation fractionnée du sel diastéréomère de son solvant approprié et libération ultérieure de la base, on prépare les esters benzy liques énantiomères (+)-1,4-dihydro-5-isopropoxy-2-méthyl-4-(2-trifluorométhyl-phényl)-1,6-naphtyridine-3-carboxylate de benzyle et (-)-1,4-dihydro-5-isopro poxy-2-méthyl-4-(2-trifluoromethyl-phènyl)-1,6-naph tyridine-3-carboxylate de benzyle et, à partir de ceux-ci soit:
a) par hydrogénation catalytique, on obtient les acides carboniques énantiomères correspondants qui, après transformation en le chlorure d'acide, sont estérifiés par l'éthanol, ou
b) les composés optiquement actifs recherchés sont directement transestérifiés par l'éthanol.

2. Procédé selon la revendication 1, pour l'obtention de (+)-1,4-dihydro-5-isopropoxy-2-méthyl-4-(2-trifluorométhyl-phényl)-1,6-naphtyridine-2-(N-méthyl-N-phénylméthyl-amino)-3-carboxylate d'éthyle et (-)-1,4-dihydro-5-isopropoxy-2-méthyl-4-(2-trifluorométhyl-phényl)-1,6-naphtyridine-2-(N-méthyl-N-phénylméthyl-amino)-3-carboxylate d'éthyle, caractérisé en ce que, pour l'estérification dans l'étape B), a), ou pour la transestérification dans l'étape B), b), on utilise le 2-(N-benzyl-N-méthylamino)éthanol.
